# EUROPÄISCHE PATENTSCHRIFT

(11) **EP 2 146 675 B1**
(45) Veröffentlichungstag und Bekanntmachung des Hinweises auf die Patenterteilung: **22.09.2010**
(21) Anmeldenummer: 08758725.9
(22) Anmeldetag: 23.05.2008
(51) Int. Cl.: A61F 13/15

(54) **VERFAHREN ZUR HERSTELLUNG EINER WINDEL**
METHOD FOR THE PRODUCTION OF A DIAPER
PROCÉDÉ DE PRODUCTION D'UNE COUCHE

(30) Priorität: 24.05.2007 DE 102007024180
(43) Veröffentlichungstag der Anmeldung: 27.01.2010
(73) Patentinhaber: Paul Hartmann AG, 89522 Heidenheim (DE)
(72) Erfinder: HORNUNG, Fridmann, Penalolén, Santiago (CL); KESSELMEIER, Rüdiger, 89542 Herbrechtingen (DE); OSTERTAG, Wolfgang, 89547 Gerstetten (DE)
(74) Vertreter: Friz, Oliver
(86) Internationale Anmeldenummer: PCT/EP2008/004131
(87) Internationale Veröffentlichungsnummer: WO 2008/141834

(56) Entgegenhaltungen:
- EP-A- 1 269 949
- EP-A- 1 428 487
- WO-A-03/082168
- WO-A-03/094815
- WO-A-2005/094746
- WO-A-2005/102241
- DE-A1-102005 048 868
- US-A- 5 622 581
- US-A1- 2002 138 056

## Beschreibung

Die Erfindung betrifft ein Verfahren zum Herstellen einer Windel, mit einem Hauptteil, bestehend aus einem Vorderbereich, einem Rückenbereich und einem in Längsrichtung dazwischen liegenden zwischen den Beinen eines Benutzers zu liegen kommenden Schrittbereich, wobei der Hauptteil einen Saugkörper umfasst, und mit beidseits an den Rückenbereich und/oder beidseits an den Vorderbereich angefügten Seitenabschnitten, welche sich in Querrichtung über seitliche Längsränder des Hauptteils hinaus erstrecken und den Vorderbereich und den Rückenbereich im angelegten Zustand der Windel miteinander verbinden, wobei die Seitenabschnitte zumindest auf der dem Schrittbereich zugewandten Seite schräg zur Längsrichtung verlaufend oder konturiert ausgebildet sind, wobei die Windel in ihrer Längsrichtung gefertigt wird und zur Erzielung des schrägen Verlaufs oder der Konturierung der Seitenabschnitte Materialaussparungen bei den Seitenabschnitten gebildet werden.

Vorstehende Windeln sind bekannt und beispielsweise in WO 2005/102241 A1 beschrieben. Sie finden häufig als verhältnismäßig ausladende Inkontinenzartikel für Erwachsene Verwendung. Es kann denkbar und wünschenswert sein, dass sowohl an den Vorderbereich als auch an den Rückenbereich des Windelhauptteils, welcher den Saugkörper umfasst, vorstehend genannte in der Querrichtung bzw. in Hüftumfangsrichtung erstreckte Seitenabschnitte angefügt sind. Genauso denkbar ist es, dass die Seitenteile nur an den Vorderbereich oder nur an den Rückenbereich des Hauptteils angefügt sind, nämlich beispielsweise dann, wenn die betreffenden Seitenteile in Hüftumfangsrichtung um den Benutzer herumgelegt und dann auf sich selbst geschlossen werden und somit eine Art Gürtel der Windel bilden.

Da Windeln oder im Speziellen Inkontinenzartikel der eingangs genannten Art in Hüftumfangsrichtung sehr ausladende Seitenabschnitte aufweisen können, gestaltet sich die Herstellung dieser Produkte, insbesondere quer zu dieser Hüftumfangsrichtung, also in Produktlängsrichtung, in schnelllaufenden Herstellungsmaschinen sehr schwierig, da die Seitenabschnitte bildende Materialien in der Herstellungsmaschine bei hohen Geschwindigkeiten nicht oder nur unter hohem Aufwand korrekt geführt werden können. Das Problem des "Aufflatterns" von insbesondere vereinzelten oder zur Maschinenrichtung hinterschnittenen Abschnitten in der Herstellungsmaschine erweist sich als problematisch.

Der vorliegenden Erfindung liegt die Aufgabe zugrunde, ein Verfahren zum Herstellen einer Windel der eingangs genannten Art anzugeben, mit dem die vorstehend geschilderten Probleme der Konfigurierung und Anfügung von Seitenabschnitten besser beherrschbar sind.

Diese Aufgabe wird bei einem Verfahren der genannten Art erfindungsgemäß dadurch gelöst, dass die beidseitigen Seitenabschnitte bildende Materialbahn in der Längsrichtung gefördert wird; dass die Materialaussparungen zur Erzielung des schrägen Verlaufs oder der Konturierung der Seitenabschnitte durch Anbringen einer Öffnung mit einem durchgehenden Umfangsrand in der Materialbahn gebildet werden; dass danach die Materialbahn in der Längsrichtung in Teilbahnen geteilt wird und die Teilung die Öffnung durchläuft; dass von den beiden Teilbahnen jeweils quer zur Längsrichtung Abschnitte abgetrennt werden, um die beidseits zu applizierenden Seitenabschnitte zu bilden und dass die Abschnitte an den jeweiligen Rückenbereich und/oder an den Vorderbereich angefügt werden.

Es wird also in die in der Längsrichtung endlos geführte Materialbahn, aus der die Seitenabschnitte der Windel gebildet werden, eine allseits umgebene Öffnung eingebracht, insbesondere ausgeschnitten bzw. ausgestanzt. Diese Öffnung bildet dann nach der Teilung den schrägen Verlauf oder die Konturierung der Seitenabschnitte an ihrer dem Schrittbereich der Windel zugewandten Seite. Die Seitenabschnitte begrenzen dann auch zusammen mit dem Schrittbereich des Windelhauptteils die Beinöffnungen der Windel. Das erfindungsgemäße Verfahren erweist sich insoweit als vorteilhaft, als durch das Anbringen der genannten Öffnung die Materialbahn in der Längsrichtung bzw. Maschinenrichtung immer noch kontinuierlich durchgehend und an den Außenrändern hinterschnittfrei erstreckt ist. Insofern kann die Bahn ohne Probleme geführt werden. Auch wenn die Materialbahn in der Längsrichtung geteilt wird, wobei ein Trenn- oder Teilungsschnitt die Öffnung, vorzugsweise symmetrisch bzw. hälftig durchläuft, bleibt die Materialbahn bzw. die nach der Längsteilung entstehenden Teilbahnen in der Längsrichtung bzw. Maschinenrichtung durchgehend, bis sie dann quer zur Längsrichtung in zu applizierende Seitenabschnitte vereinzelt wird.

Es sei an dieser Stelle auch darauf hingewiesen, dass nach der Längsteilung der Materialbahn die hierbei gebildeten beiden Teilbahnen zunächst endlos auf eine Rolle aufgewickelt und für eine spätere Verwendung zwischengespeichert werden könnten. Für die weitere Verwendung und Applizierung würden dann die endlos aufgewickelten Teilbahnen wieder abgewickelt und an den Vorderbereich und/oder Rückenbereich des in Längsrichtung geförderten Windelhauptteils angefügt. Auch dies wäre als vom Erfindungsgedanken erfasst anzusehen.

Gemäß einem weiteren Erfindungsgedanken werden die durch Teilung der Materialbahn in der Längsrichtung resultierenden Teilbahnen einander überkreuzend geführt, so dass ein Seitenwechsel in Bezug auf den Hauptteil herbeigeführt wird und die geteilten Öffnungen der beiden Teilbahnen dann jeweils nach außen weisen. Es sei aber ausdrücklich darauf hingewiesen, dass das erfindungsgemäße Verfahren auch ohne diese besonders vorteilhafte Verfahrensvariante ausführbar ist.

Insbesondere bei Windeln mit in Hüftumfangsrichtung sehr ausladenden Seitenabschnitten erweist es sich als vorteilhaft, wenn die Materialbahn beidseits wenigstens um eine in Längsrichtung verlaufende Falzlinie auf sich selbst gefaltet wird. In vorteilhafter Weise wird die Materialbahn beidseits um wenigstens zwei Faltlinien, insbesondere um drei oder vier Faltlinien, auf sich selbst gefaltet.

Diese Faltung der Materialbahn um eine in Längsrichtung verlaufende Falzlinie ist insbesondere dann vorteilhaft, wenn die Erstreckung des jeweiligen Seitenabschnitts im entfalteten Zustand in Querrichtung, also senkrecht zur Längsrichtung, über den Längsrand des Hauptteils hinaus wenigstens 10 cm, insbesondere wenigstens 15 cm, insbesondere wenigstens 18 cm und weiter insbesondere wenigstens 20 cm beträgt.

Weiter erweist es sich als vorteilhaft, wenn auf sich selbst gefaltete und flächenhaft aneinander anliegende Teilbereiche der Seitenabschnitte in dieser gefalteten Konfiguration lösbar aneinander fixiert werden, so dass ein Aufflattern in der Herstellungsmaschine weiter verhindert werden kann. Allerdings werden die mit ihren Teilbereichen aufeinander gefalteten Materialabschnitte vorzugsweise derart lösbar fixiert, dass beim Entfalten durch einmaliges Ziehen an den Seitenabschnitten die lösbare Fixierung ohne wesentlichen Widerstand auftrennbar ist. Für diesen Zweck erweist es sich insbesondere als vorteilhaft, wenn die lösbare Fixierung der aufeinander gefalteten Teilbereiche der Seitenabschnitte durch thermisch und/oder Ultraschall erzeugte Fügestellen ausgebildet ist. Es wäre jedoch hiervon abweichend oder zusätzlich auch eine Verbindung mittels Haftkleber denkbar und vorteilhaft.

Vorteilhafterweise werden die aufeinander gefalteten Teilbereiche der Seitenabschnitte derart lösbar aneinander fixiert, dass ein Anfassbereich der Seitenabschnitte unfixiert bleibt und mit den Fingern eines Benutzers oder einer Pflegeperson gut greifbar ist. Insbesondere werden die Seitenabschnitte derart gefaltet, dass dieser Anfassbereich in Querrichtung über die gefaltete Konfiguration vorsteht.

Insbesondere bei Seitenabschnitten mit verhältnismäßig großer Erstreckung in Umfangsrichtung erweist es sich als vorteilhaft, wenn die Materialbahn derart quer zur Längsrichtung geteilt wird, dass die Seitenabschnitte in Längsrichtung im Bereich der Anfügung an den Hauptteil eine Längserstreckung von wenigstens 10 cm, insbesondere von wenigstens 14 cm, insbesondere von wenigstens 18 cm und weiter insbesondere von wenigstens 22 cm haben.

In Weiterbildung der Erfindung von besonderer Bedeutung wird vorgeschlagen, dass vor dem Teilen der Materialbahn in der Längsrichtung Verschlussmittel auf die Materialbahn aufgebracht und dort festgelegt werden. Diese Verschlussmittel dienen dem Schließen der Windel in Hüftumfangsrichtung, wenn die Windel durch den Benutzer oder durch Pflegepersonen angelegt wird. Es kann sich hierbei um an sich beliebige klebend haftende oder mechanisch wirkende (z. B. durch
Haken-/Schlaufenmaterialien) Verschlussmittel handeln, die bei der Herstellung der Windel vorzugsweise ebenfalls ausgehend von endlosen Bahnmaterialien zugeführt, quer zur Förderrichtung abgetrennt und dann auf die herzustellende Windel appliziert und dort festgelegt werden (cut-and-place-Verfahren).

Es erweist sich dabei weiter als vorteilhaft, wenn die Verschlussmittel nahe oder im Bereich der Teilung in Längsrichtung aufgebracht werden. Dies ist herstellungstechnisch deshalb vorteilhaft, weil die jeweiligen den beiden Seitenabschnitten (links/rechts) zugeordneten Verschlussmittel bei dem erfindungsgemäßen Verfahren nicht wie beim Stand der Technik links und rechts außen, sondern nahe bei einander appliziert werden, was den verfahrensmäßigen Aufwand weiter herabsetzt. In Weiterbildung dieses Erfindungsgedankens werden die Verschlussmittel so aufgebracht, dass sie den Verlauf der anschließenden Teilung überbrücken und im Zuge dieser Teilung ebenfalls durchtrennt werden. Nach dieser Erfindungsvariante bildet also ein Abschnitt eines betreffenden Verschlussmittelsubstrats die beidseitigen Verschlussmittel. Dies stellt eine weitere Vereinfachung des Verfahrens und der Vorrichtung zu seiner Durchführung dar.

Obschon die in der Materialbahn auszubildende Öffnung an sich beliebig ausgebildet sein kann, also insbesondere rund, oval oder rechteckig ausgebildet sein kann, erweist es sich als vorteilhaft, wenn die Öffnung mit einer verrundeten, vorzugsweise ungefähr ovalen Kontur ausgebildet wird, die sich dann in einem verrundeten Verlauf der späteren Seitenabschnitte wiederfindet.

Nach einer grundsätzlichen Verfahrensvariante wäre es denkbar und vorteilhaft, dass die Windeln derart gefertigt werden, dass bei in der Längsrichtung aufeinander folgend geförderten Windeln der Rückenbereich der einen Windel an den Rückenbereich der anderen Windel anschließt und der Vorderbereich der einen Windel an den Vorderbereich der anderen Windel anschließt. Alternativ hierzu wäre es denkbar, dass der Rückenbereich der einen Windel an den Vorderbereich der anderen Windel anschließt.

Ungeachtet dessen erweist es sich als vorteilhaft, wenn die Abtrennung der Abschnitte von der Materialbahn bzw. von deren Teilbahnen quer zur Längsrichtung derart ausgeführt wird, dass jeweils ein quer zur Längsrichtung abgetrennter Abschnitt Seitenteile von zwei aufeinander folgend geförderten Windeln bildet. Dies wäre dann ein Rückenbereich und ein Rückenbereich oder ein Vorderbereich und ein Vorderbereich oder jeweils Rückenbereich angrenzend an Vorderbereich der aufeinander folgend geförderten Windeln. Solchenfalls erfolgt dann die Vereinzelung der Seitenabschnitte von aufeinander folgend geförderten Windeln gleichzeitig mit der Vereinzelung der Windeln von einer in Längsrichtung durchgehenden Bahn.

Die Materialbahn und damit die den Windelhauptteil angefügten Seitenabschnitte sind vorzugsweise aus einem Vliesstoff gebildet, insbesondere und vorzugsweise können Spunbond-Materialien (S) oder Spunbond-Meltblown-Materialien (SM), oder beidseitig mit Spunbond-Materialien versehene Meltblown-Schichten (SMS) oder auch kardierte Vliesmaterialien Verwendung finden. Auch Vliesstofflaminate, also insbesondere zweilagige, dreilagige oder mehrlagige Kombinationen der vorgenannten Vliesstoffe, können Verwendung finden. Die Verbindung der einzelnen Lagen kann durch an sich übliche und bekannte Verfahren, beispielsweise durch thermische Fügeverfahren (Verschweißung, insbesondere Laserverschweißung) oder durch Ultraschallschweißverfahren erfolgen; auch die kalte Verpressung, Vernadelung, Vernähung oder Verklebung von Vliesstoffmaterialien ist denkbar. Vorzugsweise werden die seitlich an den Windelhauptteil angefügten Seitenabschnitte zumindest abschnittsweise atmungsaktiv ausgebildet, wobei insbesondere eine Mikroporosität als vorteilhaft angesehen wird, die sowohl einen Luftaustausch als auch eine Durchlässigkeit für Feuchtigkeit in Form von Wasserdampf gestattet. Die Seitenabschnitte haben in vorteilhafter Weise ein Flächengewicht von 10 bis 150 g/m², insbesondere 15 - 100 g/m², weiter insbesondere 20 - 50 g/m².

Es kann sich weiter als vorteilhaft erweisen, wenn die Seitenabschnitte von Vorderbereich und Rückenbereich verschieden ausgebildet werden. Sie können sich beispielsweise hinsichtlich der Art, Beschaffenheit oder Anordnung von Verschlussmitteln unterscheiden, oder sie können sich hinsichtlich des Materials und/oder des Flächengewichts und/oder der Atmungsaktivität der verwendeten Materialbahn unterscheiden. Im letzten Fall wird zur Herstellung der Seitenabschnitte im Vorderbereich und im Rückenbereich jeweils eine endlose Materialbahn der Herstellungsvorrichtung zugeführt und erfindungsgemäß verarbeitet.

Es hat sich als vorteilhaft erwiesen, wenn eine die Hauptteile bildende Hauptteilbahn mit einer Geschwindigkeit v₁ von 100 - 600 m/min, insbesondere von 110 - 500 m/min und weiter insbesondere von 120 - 400 m/min gefördert wird.

Desweiteren hat sich als vorteilhaft erwiesen, wenn die die Seitenabschnitte bildende Materialbahn mit einer Geschwindigkeit v2 von 50 - 300 m/min, insbesondere von 55 - 250 m/min und weiter insbesondere von 60 - 200 m/min gefördert wird.

Als besonders vorteilhaft erweist es sich, wenn das Verhältnis von v₂ zu v₁ 0,25 - 0,75, insbesondere 0,30 - 0,65, insbesondere 0,35 - 0,65, insbesondere 0,40 - 0,60 und weiter insbesondere 0,45 - 0,55 beträgt.

Weitere Merkmale, Einzelheiten und Vorteile der Erfindung ergeben sich aus den beigefügten Patentansprüchen und aus der zeichnerischen Darstellung und nachfolgenden Beschreibung einer Ausführungsform des erfindungsgemäßen Verfahrens und einer hierdurch gefertigten Windel. In der Zeichnung zeigt:
- Figur 1: eine schematische Darstellung einer mit dem erfindungsgemäßen Verfahren herzustellenden Windel als Draufsicht;
- Figuren 2a bis c: verschiedene Faltzustände der Windel nach Figur 1;
- Figur 3a: eine schematische Darstellung des erfindungsgemäßen Herstellverfahrens, wobei die Förderung einer die Seitenabschnitte bildenden Materialbahn dargestellt ist;
- Figur 3b: eine schematische Darstellung des erfindungsgemäßen Herstellverfahrens, wobei die Förderung einer die Hauptteile bildenden Hauptteilbahn dargestellt ist;
- Figur 4: eine schematische Darstellung der Anbringung der Seitenabschnitte an die Windel;
- Figur 5: eine schematische Schnittansicht durch eine Materialbahn mit aufgebrachten Verschlussmitteln nach einer Ausführungsform; und
- Figur 6: eine schematische Schnittansicht durch eine Materialbahn mit aufgebrachten Verschlussmitteln nach einer anderen Ausführungsform.

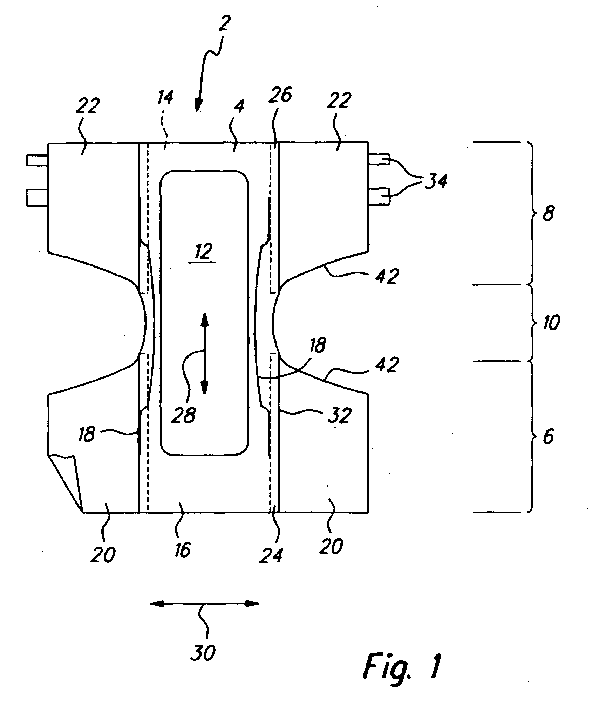

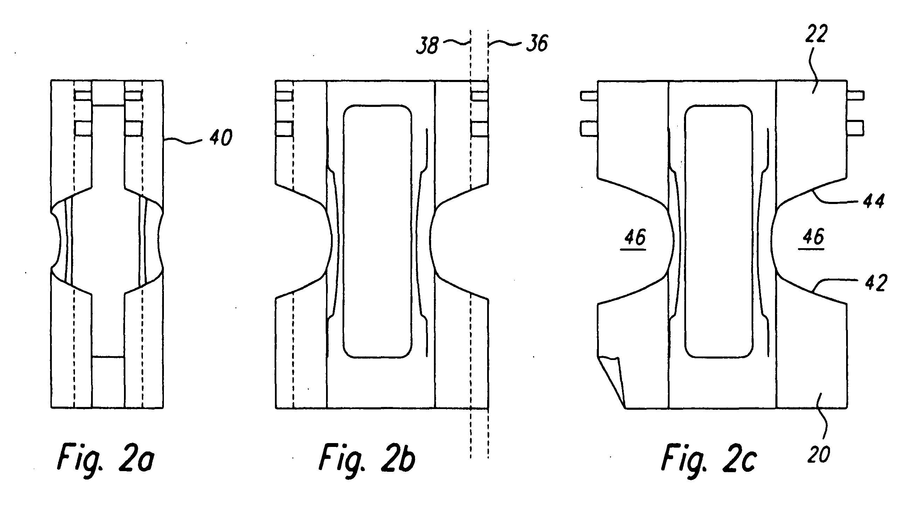

Die Figuren 1 und 2 zeigen die auf erfindungsgemäße Weise herzustellende Windel 2 in verschiedenen Faltzuständen. Die Windel 2 umfasst einen insgesamt mit dem Bezugszeichen 4 bezeichneten Windelhauptteil, der häufig auch als Chassis bezeichnet wird. Der Hauptteil 4 umfasst einen Vorderbereich 6, einen Rückenbereich 8 und einen dazwischen liegenden Schrittbereich 10, der zwischen den Beinen eines Benutzers zu liegen kommt, wenn die Windel 2 an einen Benutzer angelegt ist. Der Hauptteil 4 umfasst auch einen Saugkörper 12, der zur Aufnahme und dauerhaften Speicherung von Körperflüssigkeiten in geeigneter Weise dimensioniert ist. Der Saugkörper 12 ist üblicherweise von einer flüssigkeitsundurchlässigen Schicht 14 unterfangen, welche auch die äußere Sichtseite des Inkontinenzartikels bilden kann. Ferner ist der Saugkörper 12 üblicherweise von einer flüssigkeitsdurchlässigen Schicht 16 (Topsheet) überfangen. Ferner dargestellt sind beidseits des Saugkörpers 12 verlaufende elastifizierende Elemente 18, die unter Spannung stehende vorzugsweise elastisch dehnbare Materialien umfassen können. Insbesondere kann der Hauptteil 4 seitliche aufstehende Barrieremittel umfassen, welche einen Seitenauslaufschutz bilden und den Saugkörper flankieren. Diese aufstehenden Barrieremittel umfassen ebenfalls unter elastischer Vorspannung stehende Elastifizierungsmittel. Die vorstehend beschriebenen Komponenten der Windel 2 bilden den Hauptteil 4 bzw. sind dem Hauptteil 4 zuzuordnen.

Die Windel 2 umfasst des Weiteren beidseits an den Vorderbereich 6 und an den Rückenbereich 8 unlösbar angefügte Seitenabschnitte 20 und 22. Die Seitenabschnitte 20, 22 sind an Längsrandabschnitte 24 und 26 des Hauptteils 4 unlösbar angefügt. Die Seitenabschnitte 20, 22 erstrecken sich in einer zu einer Längsrichtung 28 der Windel 2 senkrechten Querrichtung 30 in Hüftumfangsrichtung über einen Längsseitenrand 32 des Hauptteils 2 hinaus. Die Seitenabschnitte 20, 22 werden beim Anlegen der Windel 2 in Hüftumfangsrichtung um den Benutzer herumgelegt und in der Regel aufeinander geschlossen. Zum Fixieren der vorderen und hinteren Seitenabschnitte 20, 22 aneinander sind Verschlussmittel 34 an sich bekannter und daher nicht näher beschriebener Art vorgesehen, die im beispielhaft dargestellten Fall an den Seitenabschnitten 22 des Rückenbereichs 8 angedeutet sind.

Es wäre auch denkbar, dass die Windel 2 nur im Vorderbereich 6 oder nur im Rückenbereich 8 Seitenabschnitte aufweist, die dann aber in der Querrichtung 30 ausladender als vorstehend skizziert ausgebildet sind, um in Hüftumfangsrichtung auf sich selbst oder auf den Hauptteil 4 der Windel 2 geschlossen zu werden.

Wie aus den Figuren 2a bis 2c ersichtlich, sind die jeweiligen Seitenabschnitte 20, 22 in der Längsrichtung 38 auf sich selbst gefaltet, so dass eine Z-förmige oder leporelloförmige Faltung entsteht. Diese gefaltete Konfiguration ist dann, wie in Figur 2a dargestellt, um eine weitere Falzlinie 40, die ungefähr entlang des Längsseitenrands 32 des Hauptteils 4 verläuft, auf den Hauptteil 4 gefaltet.

Man erkennt aus den Figuren 1 und 2a bis 2c, dass die Seitenabschnitte 20, 22 auf ihrer dem Schrittbereich 10 zugewandten bzw. den Schrittbereich 10 teilweise begrenzenden Seite schräg zur Längsrichtung 28 verlaufen. Ein Rand 42 bzw. 44 der Seitenabschnitte 20, 22 verläuft also nicht exakt in der Querrichtung 30, sondern schräg hierzu bzw. zur Längsrichtung 28, so dass eine vorzugsweise bogenförmig verlaufende Kontur gebildet ist. Der Rand 42 oder 44 könnte aber auch gerade, jedoch schräg zur Längsrichtung 28 bzw. Querrichtung 30 verlaufen, so dass eine Keilform der Seitenabschnitte und der Beinöffnungen 46 gebildet ist.

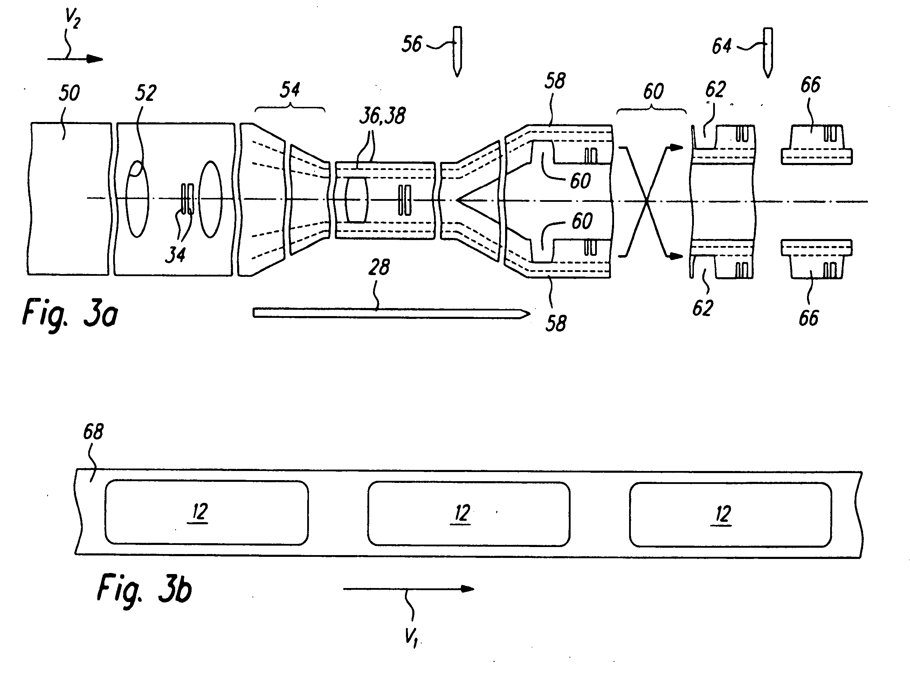

Für die Herstellung dieser Windel 2 erweist es sich als vorteilhaft, wenn eine die Seitenabschnitte 20, 22 bildende Materialbahn in Längsrichtung endlos zugeführt wird. Solchenfalls werden die Ränder 42, 44 durch Schnittvorgänge gebildet, d. h. es werden Materialaussparungen, welche dann dem Schrittbereich 10 bzw. den Beinöffnungen 46 der herzustellenden Windel 2 werden, an der Materialbahn vorgesehen. Das hierfür anwendbare erfindungsgemäße Verfahren wird nun anhand der Figur 3 beschrieben:

Figur 3a zeigt schematisch das Zuführen und Konfigurieren einer Materialbahn 50, aus der die vorderen und/oder hinteren Seitenabschnitte 20, 22 der Windel 2 gebildet werden. Figur 3b zeigt schematisch das Zuführen einer die Windelhauptteile 4 bildenden Hauptteilbahn 68 mit einer Geschwindigkeit v₁. Die Materialbahn 50 wird in der Längsrichtung 28 (Längsfertigung) mit einer Geschwindigkeit v₂ gefördert. Zur Bildung der späteren Konturierung der Seitenabschnitte 20, 22 auf ihrer dem Schrittbereich 10 zugewandten Seite wird in der Materialbahn 50 eine Öffnung 52 ausgeschnitten, und zwar derart, dass diese Öffnung 52 in der Ebene der Materialbahn 50 voll umfänglich umschlossen ist. Vor oder nach dem Ausbilden der Öffnung 52 werden im beispielhaft dargestellten Fall Verschlussmittel 34 in der Fertigungsrichtung zwischen zwei aufeinander folgenden Öffnungen 52 appliziert. Es handelt sich hierbei um an sich bekannte klebend und/oder mechanisch haftende Verschlussmittel 34, beispielsweise in Form von streifenförmigen Verschlusstapes.

Die Materialbahn 50 wird in einer Faltstation 54 beidseits von außen um mehrere in der Längsrichtung 28 verlaufende Falzlinien 36, 38 auf sich selbst nach innen gefaltet, so dass sich eine Z- oder leporelloförmige Faltung ergibt. In einer Trennstation 56 wird die Materialbahn 50 in der Längsrichtung 28 geteilt, so dass zwei Teilbahnen 58 in der Längsrichtung 28 weitergefördert werden.

In einer Kreuzungsstation 60 werden die Teilbahnen 58 einander überkreuzend geführt, so dass ein Seitenwechsel in Bezug auf die Fertigungsrichtung bzw. in Bezug auf eine in der Figur 3 nicht dargestellte Hauptteilbahn herbeigeführt wird. Die Teilung in Längsrichtung erfolgt dabei derart, dass der Trennvorgang vorzugsweise mittig oder symmetrisch durch die Öffnungen 52 verläuft, so dass randoffene Materialaussparungen 62 gebildet werden.

Man erkennt, dass die randoffenen Materialaussparungen 62 nun jeweils voneinander weg nach außen weisen.

In einer Vereinzelungsstation 64 werden die in Längsrichtung immer noch kontinuierlichen Teilbahnen 58 quer zur Längsrichtung 28 in Abschnitte 66 getrennt. Diese Abschnitte 66 können dann auf die in Figur 3b dargestellte Hauptteilbahn 68 appliziert und dort an Längsrandabschnitten 24, 26 des späteren Windelhauptteils 4 unlösbar befestigt werden.

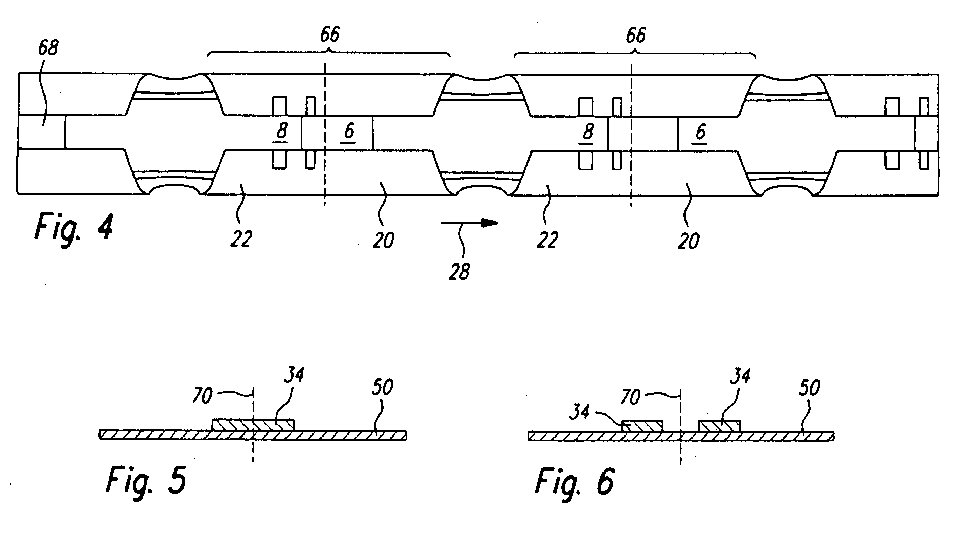

Bei den in Figur 3a schematisch dargestellten Abschnitten 66 nach der Abtrennung von den endlosen Teilbahnen 58 kann es sich entweder um einen einer einzigen Windel zuzuordnenden Seitenabschnitt handeln, oder es wäre denkbar, dass dieser Abschnitt Seitenabschnitte zweier in der Längsrichtung 28 bzw. Produktionsrichtung aufeinanderfolgend geförderter Windeln bildet. Die Seitenabschnitte würden im letzteren Fall dann vorzugsweise und zweckmäßigerweise mit der schlussendlichen Vereinzelung der Windeln von einer endlosen Bahn ebenfalls quer zur Längsrichtung abgetrennt werden. Ein solcher Prozess ist in Figur 4 schematisch dargestellt. Man erkennt, dass Abschnitte 66 zur Bildung von Seitenabschnitten 20, 22 an eine endlose Hauptteilbahn 68, welche die Windelhauptteile 4 bildet, angefügt und nach innen gefaltet sind. Jeder Abschnitt 66 umfasst jeweils die Seitenabschnitte 20, 22 zweier in der Längsrichtung 28 bzw. Produktionsrichtung aufeinanderfolgend geförderter Windeln. Bei dem Prozess gemäß Figur 4 werden die Windeln derart gefertigt, dass bei in der Längsrichtung 28 aufeinander folgend geförderten Windeln der Rückenbereich 8 der einen Windel an den Vorderbereich 6 der anderen Windel anschließt.

Bevorzugte Bahngeschwindigkeiten v₁ der Hauptteilbahn 68 und v₂ der die Seitenabschnitte 20, 22 bildenden Materialbahn 50 ergeben sich aus den Patentansprüchen 13 - 15.

Es wäre auch denkbar, dass in einen Herstellungsprozess einer Windel zwei der in Figur 3a dargestellten Konfigurationslinien implementiert werden, wobei eine Linie die Seitenabschnitte des Vorderbereichs und die andere Linie die Seitenabschnitte des Rückenbereichs der Windel bereitstellt. Auf diese Weise können dann auch verschiedene Bahnmaterialien zur Bildung der Seitenabschnitte im Vorderbereich und im Rückenbereich eingesetzt werden. Es wurde eingangs bereits darauf hingewiesen, dass der Schritt des Überkreuzens der Teilbahnen 58 nicht zwingend erforderlich ist. Die Seitenabschnitte könnten auch in der Anordnung vor der Kreuzungsstation 62 auf eine Hauptteile fördernde Bahn appliziert und dort festgelegt und dann - wenn erforderlich - nach außen umgeklappt werden.

Durch die erfindungsgemäße Verfahrensführung wird erreicht, dass die Materialbahn 50 bzw. die Teilbahnen 58 nach der Längsteilung in der Produktionsrichtung kontinuierlich endlos gefördert werden können und dass randseitig offene Materialaussparungen 62 erst nach der Längsteilung der Materialbahn 50 auftreten. Bis dahin sind die Öffnungen 52 allseitig umgeben und können daher lagestabil transportiert werden, ohne dass es zu einem störenden Aufflattern der Materialien kommt. Bei der erfindungsgemäßen Verfahrensführung ist derjenige Fertigungsabschnitt, entlang dessen mit randoffenen Materialaussparungen 62 gearbeitet wird, gegenüber dem Stand der Technik minimiert. Hierin liegt eine wesentliche Verbesserung des Herstellverfahrens. Des Weiteren erweist es sich als vorteilhaft, dass Verschlussmittel 34 für die beidseitigen Seitenabschnitte in einem aufgebracht werden können. Die Längsteilung erfasst, wie vorausgehend beschrieben, dann auch diese Verschlussmittel 34. Dies ist schematisch in Figur 5 dargestellt, wobei die Zeichnungsebene senkrecht zur Fertigungsrichtung verläuft und die unterbrochene Linie in Figur 5 eine Längsteilungsebene 70 der Trennstation 56 bezeichnet. Figur 6 zeigt schematisch die Positionierung von Verschlussmitteln 34 auf der Materialbahn 50 im Bereich der Längsteilungsebene 70, wobei die Verschlussmittel 34 im Unterschied zu Figur 5 die Längsteilungsebene 70 nicht überbrücken.

## Patentansprüche

1. Verfahren zum Herstellen einer Windel (2), mit einem Hauptteil (4), bestehend aus einem Vorderbereich (6), einem Rückenbereich (8) und einem in Längsrichtung (28) dazwischen liegenden zwischen den Beinen eines Benutzers zu liegen kommenden Schrittbereich (10), wobei der Hauptteil (4) einen Saugkörper (12) umfasst, und mit beidseits an den Rückenbereich (8) und/oder beidseits an den Vorderbereich (6) angefügten Seitenabschnitten (20, 22), welche sich in Querrichtung (30) über seitliche Längsränder (32) des Hauptteils (4) hinaus erstrecken und den Vorderbereich (6) und den Rückenbereich (8) im angelegten Zustand der Windel miteinander verbinden, wobei die Seitenabschnitte (20, 22) zumindest auf der dem Schrittbereich (10) zugewandten Seite schräg zur Längsrichtung (28) verlaufend oder konturiert ausgebildet sind, wobei die Windel (2) in ihrer Längsrichtung (28) gefertigt wird und zur Erzielung des schrägen Verlaufs oder der Konturierung der Seitenabschnitte (20, 22) Materialaussparungen (62) bei den Seitenabschnitten (20, 22) gebildet werden, **dadurch gekennzeichnet, dass** eine die beidseitigen Seitenabschnitte (20, 22) bildende Materialbahn (50) in der Längsrichtung (28) gefördert wird; dass die Materialaussparungen (62) zur Erzielung des schrägen Verlaufs oder der Konturierung der Seitenabschnitte (20, 22) durch Anbringen einer Öffnung (52) mit einem durchgehenden Umfangsrand in der Materialbahn (50) gebildet werden; dass danach die Materialbahn (50) in der Längsrichtung (28) in Teilbahnen (58) geteilt wird und die Teilung die Öffnung (52) durchläuft; dass von den beiden Teilbahnen (58) jeweils quer zur Längsrichtung (28) Abschnitte (66) abgetrennt werden, um die beidseits zu applizierenden Seitenabschnitte (20, 22) zu bilden und dass die Abschnitte (66) an den jeweiligen Rückenbereich (8) und/oder an den Vorderbereich (6) angefügt werden.

2. Verfahren nach Anspruch 1, **dadurch gekennzeichnet, dass** die durch die Teilung in der Längsrichtung gebildeten Teilbahnen (58) einander überkreuzend geführt werden, so dass ein Seitenwechsel in Bezug auf den Hauptteil (4) herbeigeführt wird und die geteilten Öffnungen (52) als Materialaussparungen (62) in den beiden Teilbahnen (58) dann jeweils nach außen weisen.

3. Verfahren nach Anspruch 1 oder 2, **dadurch gekennzeichnet, dass** die Materialbahn (50) beidseits wenigstens um eine in Längsrichtung (28) verlaufende Falzlinie (36, 38) auf sich selbst gefaltet wird.

4. Verfahren nach Anspruch 1, 2 oder 3, **dadurch gekennzeichnet, dass** vor dem Teilen der Materialbahn (50) in der Längsrichtung (28) Verschlussmittel (34) auf die Materialbahn (50) aufgebracht und dort festgelegt werden.

5. Verfahren nach Anspruch 4, **dadurch gekennzeichnet, dass** die Verschlussmittel (34) nahe oder im Bereich einer die Längsrichtung (28) einschließenden Teilungsebene (70) aufgebracht werden.

6. Verfahren nach Anspruch 4, **dadurch gekennzeichnet, dass** die Verschlussmittel (34) den Verlauf der anschließenden Teilung überbrücken und im Zuge dieser Teilung ebenfalls durchtrennt werden.

7. Verfahren nach einem oder mehreren der vorstehenden Ansprüche, **dadurch gekennzeichnet, dass** die Öffnung (52) ungefähr oval ausgebildet wird.

8. Verfahren nach einem oder mehreren der vorstehenden Ansprüche, **dadurch gekennzeichnet, dass** die Windeln derart gefertigt werden, dass bei in der Längsrichtung (28) aufeinander folgend geförderten Windeln der Rückenbereich (8) der einen Windel an den Rückenbereich (8) der anderen Windel anschließt und der Vorderbereich (6) der einen Windel an den Vorderbereich (6) der anderen Windel anschließt.

9. Verfahren nach einem oder mehreren der vorstehenden Ansprüche, **dadurch gekennzeichnet, dass** die Windeln derart gefertigt werden, dass bei in der Längsrichtung (28) aufeinander folgend geförderten Windeln der Rückenbereich (8) der einen Windel an den Vorderbereich (6) der anderen Windel anschließt.

10. Verfahren nach einem oder mehreren der vorstehenden Ansprüche, **dadurch gekennzeichnet, dass** je ein quer zur Längsrichtung (28) abgetrennter Abschnitt (66) Seitenteile (20, 22) von zwei aufeinander folgend geförderten Windeln bildet.

11. Verfahren nach einem oder mehreren der vorstehenden Ansprüche, **dadurch gekennzeichnet, dass** die Seitenabschnitte (20, 22) von Vorderbereich (6) und Rückenbereich (8) verschieden ausgebildet sind.

12. Verfahren nach Anspruch 11, **dadurch gekennzeichnet, dass** die Seitenabschnitte (20, 22) von Vorderbereich (6) und Rückenbereich (8) sich hinsichtlich des Materials und/oder des Flächengewichts und/oder der Atmungsaktivität der verwendeten Materialbahn unterscheiden.

13. Verfahren nach einem oder mehreren der vorstehenden Ansprüche, **dadurch gekennzeichnet, dass** eine die Hauptteile (4) bildende Hauptteilbahn (68) mit einer Geschwindigkeit v₁ von 100 - 600 m/min, insbesondere von 110 - 500 m/min und weiter insbesondere von 120 - 400 m/min gefördert wird.

14. Verfahren nach einem oder mehreren der vorstehenden Ansprüche, **dadurch gekennzeichnet, dass** die die Seitenabschnitte (20, 22) bildende Materialbahn (50) mit einer Geschwindigkeit v₂ von 50 - 300 m/min, insbesondere von 55 - 250 m/min und weiter insbesondere von 60 - 200 m/min gefördert wird.

15. Verfahren nach Anspruch 13 und 14, **dadurch gekennzeichnet, dass** das Verhältnis von v₂ zu v₁ 0,25 - 0,75, insbesondere 0,30 - 0,65, insbesondere 0,35 - 0,65, insbesondere 0,40 - 0,60 und weiter insbesondere 0,45 - 0,55 beträgt.

## Claims

1. A method for the production of a diaper (2) with a main body (4), consisting of a front portion (6), a back portion (8) and a crotch portion (10) that lies in a longitudinal direction (28) between them, and is placed between the legs of a wearer, wherein the main body (4) comprises an absorbent body (12), and with lateral portions (20, 22) that are attached on both sides to the back portion (8) and/or to the front portion (6), said lateral portions (20, 22) extending in the transverse direction (30) beyond lateral longitudinal edges (32) of the main body (4) and connecting the front portion (6) and the back portion (8) to one another in the applied state of the diaper, wherein the lateral portions (20, 22), at least on the side facing the crotch portion (10), are configured in such a way that they run obliquely to the longitudinal direction (28) or are contoured, said diaper (2) being produced in its longitudinal direction (28), and in order to achieve the oblique course or contouring of the lateral portions (20, 22), material recesses (62) are formed at the lateral portions (20, 22), **characterized in that** a material web (50) that forms the lateral portions (20, 22) on both sides is conveyed in the longitudinal direction (28); that the material recesses (62) for achieving the oblique course or the contouring of the lateral portions (20, 22) are formed by making an opening (52) with a continuous circumferential edge in the material web (50); that subsequently, the material web (50) is divided/separated in the longitudinal direction (28) into partial webs (58), the separation running through the opening (52); that sections (66) are cut from the two partial webs (58), in each case transversely to the longitudinal direction (28) in order to form the lateral portions (20, 22) that are to be applied to both sides, and that the sections (66) are attached to the respective back portion (8) and/or front portion (6).

2. The method according to Claim 1, **characterized in that** the partial webs (58) that are formed by the division/separation in the longitudinal direction are conveyed such that they cross one another, so that there is a change of side in relation to the main body (4), the divided openings (52) then facing in each case outward as material recesses (62) in the two partial webs (58).

3. A method according to Claim 1 or 2, **characterized in that** the material web (50) is folded on both sides onto itself along at least one folding line (36, 38) that runs in a longitudinal direction (28).

4. A method according to Claim 1, 2 or 3, **characterized in that** closing means (34) are applied to the material web (50) and fixed there before dividing the material web (50) in the longitudinal direction (28).

5. The method according to Claim 4, **characterized in that** the closing means (34) are applied near to or in the region of a separation plane (70) that comprises the longitudinal direction (28).

6. The method according to Claim 4, **characterized in that** the closing means (34) bridge the course of the subsequent division, and are likewise cut through.

7. A method according to one or more of the preceding claims, **characterized in that** the opening (52) is approximately oval in shape.

8. A method according to one or more of the preceding claims, **characterized in that** the diapers are produced in such a way that when the diapers are consecutively conveyed in the longitudinal direction (28), the back portion (8) of one diaper follows the back portion (8) of the other diaper, and the front portion (6) of one diaper follows the front portion (6) of the other diaper.

9. A method according to one or more of the preceding claims, **characterized in that** the diapers are produced in such a way that when the diapers are consecutively conveyed in the longitudinal direction (28), the back portion (8) of one diaper follows the front portion (6) of the other diaper.

10. A method according to one or more of the preceding claims, **characterized in that** each section (66) that was cut off transversely to the longitudinal direction (28) forms lateral portions (20, 22) of two consecutively conveyed diapers.

11. A method according to one of more of the preceding claims, **characterized in that** the lateral portions (20, 22) of the front portion (6) and the back portion (8) are differently configured.

12. The method according to Claim 11, **characterized in that** the lateral portions (20, 22) of front portion (6) and the back portion (8) are different with regard to the material and/or the grammage and/or the breathability of the material web used.

13. A method according to one or more of the preceding claims, **characterized in that** the material web (68) forming the main bodies (4) is conveyed at a speed v₁ of 100-600 m/min, in particular of 110-500 m/min, and further in particular of 120-400 m/min.

14. A method according to one or more of the preceding claims, **characterized in that** the material web (50) forming the lateral portions (20, 22) is supplied at a speed v₂ of 50-300 m/min, in particular of 55-250 m/min, and further in particular of 60-200 m/min.

15. A method according to Claim 13 and 14, **characterized in that** the ratio of v₂ to v₁ is 0,25-0,75, in particular 0,30-0,65, in particular 0,35-0,65, in particular 0,40-0,60, and further in particular 0,45-0,55.

## Revendications

1. Procédé pour fabriquer une couche (2) comportant une partie principale (4), constituée d'une zone avant (6), d'une zone arrière (8) et d'une zone d'entrejambe (10) située entre les deux dans le sens longitudinal (28) venant se positionner entre les jambes d'un utilisateur, la partie principale (4) comprenant un corps absorbant (12), et comportant des segments latéraux (20, 22) joints de chaque côté de la zone arrière (8) et/ou de chaque côté de la zone avant (6), lesquels segments s'étendent dans le sens transversal (30) au-delà des bords longitudinaux latéraux (32) de la partie principale (4) et relient entre elles, à l'état posé de la couche, la zone avant (6) et la zone arrière (8), les segments latéraux (20, 22) étant réalisés inclinés par rapport au sens longitudinal (28) ou bien de façon à former un contour au moins sur le côté orienté vers la zone d'entrejambe (10), la couche (2) étant fabriquée dans son sens longitudinal (28) et, en vue d'obtenir le tracé oblique ou la forme de contour des segments latéraux (20, 22), des évidements de matière (62) étant pratiqués au niveau des segments latéraux (20, 22), **caractérisé en ce qu'**une bande de matière (50) formant les segments latéraux (20, 22) de chaque côté est amenée dans le sens longitudinal (28), **en ce que** les évidements de matière (62) en vue d'obtenir le profil oblique ou le profil de contour des segments latéraux (20, 22) sont réalisés en pratiquant une ouverture (52) avec un bord périphérique continu dans la bande de matière (50), **en ce que** la bande de matière (50) est ensuite coupée dans le sens longitudinal (28) en bandes partielles (58) et **en ce que** la séparation passe à travers l'ouverture (52), **en ce que**, à partir des deux bandes partielles (58), on coupe transversalement au sens longitudinal (28) des segments (66) afin de former les segments latéraux (20, 22) à appliquer de chaque côté et **en ce que** les segments (66) sont joints à la zone arrière (8) et/ou à la zone avant (6).

2. Procédé selon la revendication 1, **caractérisé en ce que** les bandes partielles (58) obtenues par la séparation dans le sens longitudinal sont guidées en se croisant, ce qui entraîne un changement de côté par rapport à la partie principale (4), et **en ce que** les ouvertures coupées (52) apparaissant désormais sous la forme d'évidements de matière (62) dans les deux bandes partielles (58) sont orientées vers l'extérieur.

3. Procédé selon la revendication 1 ou 2, **caractérisé en ce que** la bande de matière (50) est pliée sur elle-même de chaque côté au moins autour d'une ligne de pliage (36, 38) s'étendant dans le sens longitudinal (28).

4. Procédé selon la revendication 1, 2 ou 3, **caractérisé en ce que**, avant de couper la bande de matière (50) dans le sens longitudinal (28), des moyens de fermeture (34) sont appliqués sur la bande de matière (50) et y sont fixés.

5. Procédé selon la revendication 4, **caractérisé en ce que** les moyens de fermeture (34) sont appliqués à proximité ou dans la zone d'un plan de séparation (70) renfermant le sens longitudinal (28).

6. Procédé selon la revendication 4, **caractérisé en ce que** les moyens de fermeture (34) franchissent le tracé de la séparation à venir et sont également séparés au cours de cette séparation.

7. Procédé selon une ou plusieurs des revendications précédentes, **caractérisé en ce que** l'ouverture (52) est réalisée approximativement ovale.

8. Procédé selon une ou plusieurs des revendications précédentes, **caractérisé en ce que** les couches sont fabriquées de telle façon que, lorsque les couches sont amenées les unes à la suite des autres dans le sens longitudinal (28), la zone arrière (8) d'une couche est adjacente à la zone arrière (8) de l'autre couche et la zone avant (6) d'une couche est adjacente à la zone avant (6) de l'autre couche.

9. Procédé selon une ou plusieurs des revendications précédentes, **caractérisé en ce que** les couches sont fabriquées de telle façon que, lorsque les couches sont amenées les unes à la suite des autres dans le sens longitudinal (28), la zone arrière (8) d'une couche est adjacente à la zone avant (6) de l'autre couche.

10. Procédé selon une ou plusieurs des revendications précédentes, **caractérisé en ce que** chaque segment (66) séparé transversalement au sens longitudinal (28) forme des segments latéraux (20, 22) de deux couches qui se suivent.

11. Procédé selon une ou plusieurs des revendications précédentes, **caractérisé en ce que** les segments latéraux (20, 22) de la zone avant (6) et de la zone arrière (8) sont réalisés différents.

12. Procédé selon la revendication 11, **caractérisé en ce que** les segments latéraux (20, 22) de la zone avant (6) et de la zone arrière (8) se différencient au niveau de la matière et/ou du poids par unité de surface et/ou de l'activité de respiration de la bande de matière utilisée.

13. Procédé selon une ou plusieurs des revendications précédentes, **caractérisé en ce qu'**une bande de partie principale (68) formant la partie principale (4) est amenée à une vitesse V₁ de 100 à 600 m/min, en particulier de 110 à 500 m/min et plus particulièrement de 120 à 400 m/min.

14. Procédé selon une ou plusieurs des revendications précédentes, **caractérisé en ce que** la bande de matière (50) formant les segments latéraux (20, 22) est amenée à une vitesse V₂ de 50 à 300 m/min, en particulier de 55 à 250 m/min et plus particulièrement de 60 à 200 m/min.

15. Procédé selon les revendications 13 et 14, **caractérisé en ce que** le rapport V₂/V₁ est compris entre 0,25 et 0,75, en particulier entre 0,30 et 0,65, en particulier entre 0,35 et 0,65, en particulier entre 0,40 et 0,60 et plus particulièrement entre 0,45 et 0,55.
